# EUROPEAN PATENT APPLICATION

(11) **EP 2 724 726 A1**
(43) Date of publication of application: **30.04.2014**
(21) Application number: 14152382.9
(22) Date of filing: 30.06.2008
(51) Int. Cl.: A61K 39/39, A61P 37/04

(54) **Use of intranasally administered Pidotimod to enhance antigen-specific humoral and cell-mediated immune response**

(30) Priority: 02.07.2007 IT TO20070480
(62) Divisional of application: 08776561.6
(71) Applicant: POLICHEM S.A., 1526 Luxembourg (LU)
(72) Inventor: Caruso, Arnaldo, 20124 Brescia (IT)
(74) Representative: Pistolesi, Roberto

(57) **Abstract**

Pidotimod is used for the preparation of a medicament in a form which can be administered through the intranasal mucosal route, useful as an adjuvant in enhancing both the humoral and cell-mediated specific immune response towards specific antigen.

## Description

The present invention relates to a novel use in the therapeutic field of the active ingredient Pidotimod, and to pharmaceutical formulations comprising it.

Pidotimod, ((R)-3-[(s)-(5-oxo-2-pyrrolydinyl)]carbonyl]-thiazolidine-4-carboxylic acid), is a synthetic dipeptide provided with an in *vitro* and *in vivo* immunomodulating action.

Immune system intervenes in maintaining a homeostatic balance between our body and all foreign substances; therefore, an abnormality in this function may cause a defective response towards non-self structures, or loss of tolerance toward self antigens: in both cases, the immune system error exhibits clinically as signs of disease. Pidotimod proved to be able to exert a protective action towards infectious processes (although it is free from a direct antimicrobial and viral action), as it has been confirmed in experimental bacterial and viral infection models, and confirmed by a number of controlled clinical trials.

Particularly, Pidotimod increases the lymphocyte proliferation and the production of pro-inflammatory cytokines by pre-activated lymphocytes withdrawn from older subjects. Furthermore, it is able to increment the release of antiviral molecules (IFN-α) induced by specific stimuli (Poly I:C). *Ex vivo* studies highlighted the Pidotimod ability to increment phagocytes stimulation and relative phagocyte action, as well as the cytotoxic action of NK cells.

Pidotimod, administered in tablets via oral route, proved to be able to induce, in animal models, a higher resistance to viral infections and a higher efficiency in the treatment of recurrent respiratory infections in the paediatric patient.

Recent studies demonstrate how the vaccinations through the mucosal membranes are able to enhance the body immune defences, locally -therefore at the site of entry of the external pathogen- stimulating an efficient inflammatory response.

However, the administration of the antigen molecule alone is sometimes not able to trigger an efficient inflammatory response mucosally, and this causes a partial enzymatic degradation of the molecules, which occurs locally. Therefore, co-administration of so-called auxiliary substances represents an extremely attractive, widely-used strategy, able to enhance the local and systemic immune response.

The present invention originates from an experimental research which allowed highlighting a direct action of Pidotimod on human dendritic cells, and the ability thereof to enhance the communication between such innate immune system cells and the acquired immune system cells; the results of such research further indicate that Pidotimod administered through the intranasal mucosal route acts in *vivo* as an adjuvant drug, that is, able to enhance the specific both humoral, and cell-mediated immune response towards a specific antigen.

In view of the results of such study, a first object of the invention is the use of Pidotimod for the preparation of a medicament in a form which can be administered through the intranasal mucosal route, useful as an adjuvant in enhancing both a humoral, and cell-mediated specific immune response toward a specific antigen.

In the study which has been carried out within the scope of the invention, it has been highlighted that Pidotimod, when administered instead via oral route, is not able to enhance either the local, or the systemic immune response towards a specific antigen; this observation suggests that Pidotimod acts on mucosal cells of the upper respiratory tract, triggering a specific immune response.

Particularly, Pidotimod in the form of a nasal spray can be used as a drug capable to enhance the immune responses mucosally and systemically, in order to both prevent the respiratory tract infections, and to enhance immune responses during respiratory tract acute infections. Furthermore, Pidotimod can be used as an adjuvant in mucosal vaccines intra- nasally.

The preferred administration form is a solution which is suitable for an administration as a spray or an aerosol, consisting in Pidotimod and a pharmaceutically acceptable liquid carrier. Preferably, the administration form consists in Pidotimod in aqueous brine solution, optionally including preservatives, antioxidants, antiseptic agents, and/or agents promoting adhesion to the mucosal membrane, or penetration into the mucosal membrane. Typically, Pidotimod is used in a concentration from 1 mg/liter to 10 g/liter, preferably from 500 mg/liter to 5 g/liter.

The administration dose in the human being is typically of about 0.5 mg/spray (corresponding to about 0.130 mL).

Also within the scope of the invention is a pharmaceutical product in the form of a combined preparation, containing a mucosal vaccine and Pidotimod, in a form which can be administered through the intranasal mucosal route, for the concomitant, separated, or sequential use for the prevention of respiratory tract infections.

Pidotimod action, which justifies the use thereof in accordance with the invention, has been shown by the following experimental tests.

### 1-Pidotimod action on human dendritic cells

### a)Culture of human dendritic cells

Immature human dendritic cells (DCs) have been generated in *vitro* from circulating monocytes, as described in the literature (1-2). Monocytes were purified from peripheral blood by positive selection by the use of CD14-specific magnetic beads (Miltenyi Bio tec, Bergisch Gladbach, Germany), and cultured for 6 days in the presence of GM-CSF (800 LVmL) and IL-4 (500 U/mL) (R&D Systems, Minneapolis, MN, USA).

### b)In vitro maturation of dendritic cells

Immature dendritic cells were washed and cultured for 24-72 hours in the presence and absence of a suitable maturation stimulus, such as the bacterial lipopolysaccharide, or LPS, at a 1 µg/mL concentration. In the same manner, cells were stimulated with Pidotimod, at concentrations ranging between 10-0.5 µg/mL. The Pidotimod ability to induce a phenotypic maturation of the DCs was highlighted by immunofluorescence and flow cyto-fluorimetry methods, assessing and quantifying the expression of the CD80, CD83, CD86 markers on the DCs surface by using fluorochrome-conjugated monoclonal antibodies (Becton-Dickinson, Palo Alto, CA).

### c)ELISA for the quantification of TNF-α and MCP-I

The increase of cytokine synthesis is the signal of a functional maturation of DCs. The immature DCs are stimulated or not stimulated with 1 µg/mL LPS, or with 10-0.5 µg/mL Pidotimod, and the supernatant of the cell cultures collected after 24 hours from the stimulation onset. The presence of TNF-α and MCP-I was quantified in the cultural medium through ELISA assays (Endogen, Rockford, THE, USA).

### d) Proliferation of DCs-co-cultured naive CD4+ T lymphocytes

Functionalizing DCs are able to induce allogenic naive CD4+ T lymphocytes to proliferate and differentiate in T-helper-1 cells (Th-I) producing IFN-γ. Naive CD4+ T-cells were purified by negative selection using a "CD4+ T-cell isolation kit" in conjunction with CD45RO-specific beads (Miltenyi). The so-obtained lymphocytes were marked with Cell-Trace CFSE Cell proliferation kit (Molecular Probes Eugene, OR), then cultured at different cell ratios with Pidotimod-treated DCs in flat-bottom 96-well plates. The proliferative ability of CD4 lymphocytes was established after 4 days by cytofluorimetric analysis.

### e)Production of cytokines intracellularly

In order to analyze the cytochemical polarization of the CD4+ T lymphocytes, DCs and T lymphocytes were co-cultured for 6 days in the presence and absence of Pidotimod, then subjected to re-stimulation with PMA and ionomycin. The presence of IL-2, IFN-γ, and IL-4 intracellularly was analyzed by flow cytofluorimetry using fluorochrome-marked monoclonal antibodies as specific reagents (Becton-Dickinson).

*In-vitro* studies allowed highlighting a direct action of Pidotimod on human dendritic cells, and the ability thereof to enhance communication between such innate immune system cells and the acquired immune system cells. This has been carried out by *in vitro* monitoring of the maturation steps of the myeloid dendritic cells, the cytockine and chemokine activity thereof, and by studying the ability thereof to activate naive CD4 lymphocytes following a Pidotimod stimulation.

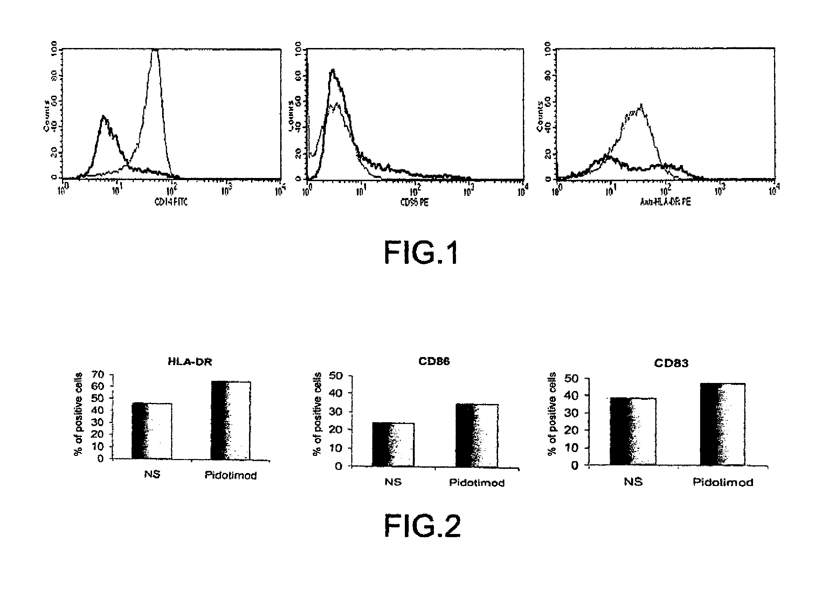

On an experimental point of view, dendritic cells were generated *in vitro* by stimulating CD14+ monocytes obtained from peripheral blood of healthy subjects for 6 days with GM-CSF + IL-4. The so-treated monocytes undergo, after 6 days, a down-regulation of the CD 14 molecule and a slight up- regulation of the HLA-DR and CD86 molecules, on the whole, getting the macroscopic appearance and the phenotype which is intrinsic of immature myeloid dendritic cells (Fig. 1).

The so-generated immature dendritic cells were stimulated with Pidotimod and with a known maturation stimulus (bacterial lipopolysaccharide, or LPS) which, since it acts through the TLR4, brings the myeloid dendritic cells derived from the monocytes to a phenotypic and functional maturation. After 24 hours, such maturation translates, in a phenotypic sense, in the expression acquisition on the cell surface of CD83, CD 86 molecules, and in the up-regulation of the HLA-DR molecules.

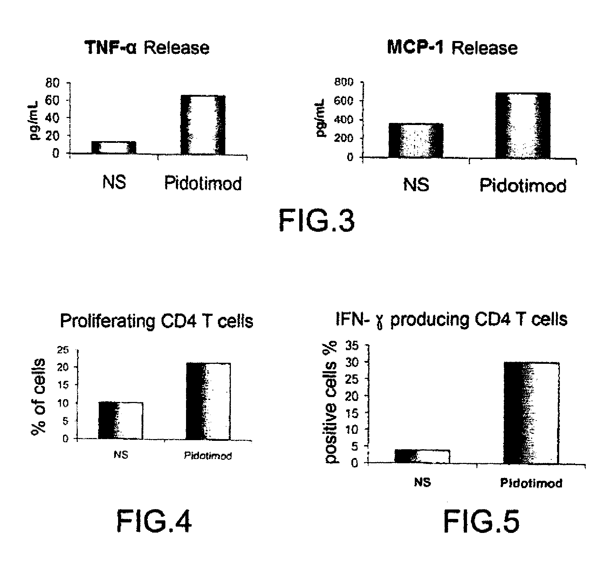

In all the examined samples, from 6 different subjects, an increase in the CD83+, CD86+, and HLA-DR+ population was noticed after 24h of maturation stimulation with Pidotimod compared to the non-treated immature dendritic cells (Fig. 2), and a concomitant increase of the pro-inflammatory molecules TNF-α and MCP-I released in the cell culture supernatant (Fig. 3).

Finally, it has been assessed whether in the context of a co-culture between dendritic cells and naive CD4 lymphocytes, Pidotimod was able to induce in the latter a higher proliferative action and an increase of the polarization in the sense of T helper- 1 with IFN-γ production. The results shown in Fig. 4 underline how dendritic cells matured in the presence of Pidotimod are able, after five days co-culture, to increment the proliferating CD4 lymphocytes percentage, and how such percentage increased also following the clonal expansion of such lymphocytes in a microenvironment rich IL-2.

After eight days, CD4+ T lymphocytes co-cultured with the dendritic cells were stimulated with PMA + ionomycin for 9 h in order to assess the acquired potentiality of T-helper-1 type. The percentage of IFN-γ-producing CD4+ T lymphocytes was significantly higher if such cells were co-cultured with dendritic cells matured in the presence of Pidotimod (Fig. 5).

These data show that Pidotimod is able to bring to phenotypic maturation myeloid dendritic cells generated in *vitro*, and to make them functional to activate the adaptative cell system in the T lymphocyte component thereof.

### 2-Action of Pidotimod as a mucosal adjuvant in the enhancement of innate immune activity

With the aim to assess the Pidotimod ability to trigger a immune response in *vivo*, such as to enhance immunogenicity of other proteins, C57BL/6 mice were immunized with the Ovalbumin (Ova) antigen mucosally (intranasally) in combination with and in the absence of Pidotimod.

### Reagents

Ovalbumin (Ova, Sigma, USA, >98% purity) is used as the antigen for both the in *vivo* and *in vitro* studies. The peptides representing the OVA dominant (Ova₂₅₇₋₂₆₄, SIINFEKL), subdominant (Ova₅₅₋₆₂, KWRFDKL) 5 and cryptic (Ova₁₁₋₁₈, CFDVFKEL) epitopes, recognized from the MHC complex of class I (H-2k^{b}) [3-5], were produced at HZI (Braunschweig, Germany).

### Animals and cell cultures

Femal C57BL/6 (H-2b) mice, 6-8 weeks of age, (Harlan-Winkelmann GmbH, Borchen, Germany) were used and treated in accordance with the guidelines of the European Community. Splenocytes were cultured in RPMI 1640 with 10% FCS, 100 LVmL penicillin, 50 µg/mL streptomycin, 5x10⁻⁵ M 2-mercaptoethanol, and 1 mM L-glutamine (GBCO BRL, Karlsruhe, Germany).

### Immunization protocol

Groups of six C57BL/6 mice (h-2^{b}), 6-8 weeks of age, were intranasally or orally immunized at days 0, 14, and 21, with 50 µg Ovalbumin (Ova) alone and in co-administration with 100 µg (intranasal) or 1 mg (oral) Pidotimod. The control mice were immunized with PBD. One day before each run (days 0, 13, 20), and a week after the last immunization (day 28), when the animals were sacrificed by CO2 inhalation, blood samples were withdrawn from the caudal vein for the determination of Ova-specific antibodies in serum.

With the aim to analyze the cell immune response, lymphocytes of splenic and medullary derivation were isolated after aseptic removal of the spleen and of the bone marrow from the femora and tibia, and the cells were subsequently suitably cultured. The experimental immunization and the followed timing are summarized in Scheme 1.

### Scheme 1

### Blood samples withdrawal (days 0, 14, 21)

Sacrifice of three mice out of each immunization group (day 28)
- Blood withdrawal;
- bronchoalveolar wash;
- intestinal wash;
- spleen withdrawal.

*In vivo* cytotoxicity experiments (one mouse out of each group) (day 42)

Sacrifice of two mice of each immunization group (day 78)
- Blood withdrawal;
- bone marrow withdrawal;
- spleen withdrawal.

### Search for anti-Ova antibodies

The dosing of anti-Ova-specific antibodies has been carried out by means of ELISA procedures. Briefly, the Ova antigen was left to be absorbed (5 µg/mL in carbonated buffer, pH 9.6) on 96-well plates (Nunc-immunoMaxiSorp, Nunc, Roskilde, Denmark) for 18 hours at 4 °C. Then, the plates were washed, and 2-fold dilutions of the animal sera were dispensed in the wells and let to rest for 1 hour at 37 °C. After the successive four washes, the biotinilated secondary anti-mouse antibody, (Sigma, Chemie, Deisenhofen, Germany) was added to each well, followed by another 1-hour incubation at 37 °C. After the successive washes, peroxidase enzyme-conjugated streptavidine (Pharmingen) was dispensed in each well. After 45 minutes, the enzymatic reaction developed through ABTS [2,2'-azino-bis(3-ethyl-benzolyn-6-sulfonic acid)] and the absorbance was read at 405 nm. The obtained results represent the mean + standard deviation for each group.

### Identification of Immunoglobulins-producing cells by means of ELISPOT assays

The total number of IgG-producing antigen-specific cells was assessed two months after the last immunization by means of ELISPOT assays. Briefly, isotype-specific antibodies (Sigma, Germany) were left to be absorbed on PVDF plates (Millipore, Bedford, USA), at 5 mg/mL concentration, or the Ova antigen (5 µg/mL in carbonated buffer, pH 9.6), in order to determine the total and antigen-specific antibodies, respectively. Then, cells deriving from spleen and bone marrow were seeded in quadruplicate at different concentrations, and let to incubate for 6 hours. After the suitable washings of the plates, the biotinilated secondary antibody (Sigma) was added for 18 hours at 4° C, then the peroxidase enzyme - conjugated streptavidine (Pharmingen) was dispensed in each well. The spots were developed by using 3-amino-9-ethylcarbazole (Sigma), analyzed by the ImmunoSpot 3 A Analyzer, and counted by the ImmunoSpot Image Analyzer software v3.2 (C.T.L.).

### Identification of interferon-γ-producing cells by means of ELISPOT assays

After one and eight weeks from the last immunization, the number of splenic and medullary interferon-γ-producing cells was assessed by ELISPOT assays. Briefly, the cells were seeded at a final concentration of 5x10⁵ and 1x10⁶/well, in quadruplicate, in the presence and absence of the restricted MCH-I peptides (SIINFEKL, KWRFDKL, CFDVFKEL). After 16 hours culture, the cells were removed, and the biotinilated anti- interferon-γ antibody was added. Then the spots were developed and analyzed as described above.

### Determination of cell-mediated in vivo cytotoxicity

With the aim of assessing cell-mediated cytotoxicity of T lymphocytes of the immunized mice, splenocytes of naive C57BL/6 mice were isolates as described above. After three sequential washes in PBS, an aliquot of cells was marked with 0.1 µM CFSE (Molecular Probes Eugene, OR), and another aliquot with 1 µM CFSE. At the middle of each aliquot, cells were pulsed for 1 hour at 37° C with the dominant, subdominant, and cryptic OVA peptides at 15 µg/mL concentration. 2x10⁷ cells were inoculated i.v. in the immunized mice, and after 16 hours the splenocytes were analyzed by cytofluorimeter. The lysis percentage was proportional to the disappearance of peptide-pulsed cells.

### Results

### Intranasally administered Pidotimod induces the production of Ova-specific IgGs in the serum

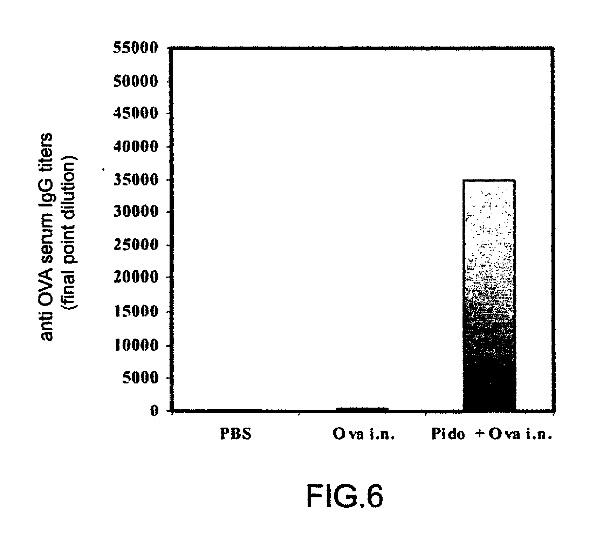

With the aim of assessing if Pidotimod was able to increment the production of OVA-specific antibodies in the serum, the animals were intranasally and orally immunized with 100 µg Pidotimod in combination with OVA. The obtained results underline how intranasally administered Pidotimod is able to highly implement the OVA immunogenic action by generating, as shown in Fig. 6, antibody titers of OVA-specific IgGs in the serum, 35-folds higher than non-Pidotimod-treated mice. Instead, oral administration of Pidotimod did not give antibody titers significantly higher than the administration with OVA alone (non-shown data).

### Differentiation of splenocytes and bone marrow cells in IFN-γ-secreting elements after intranasal immunization with Pidotimod

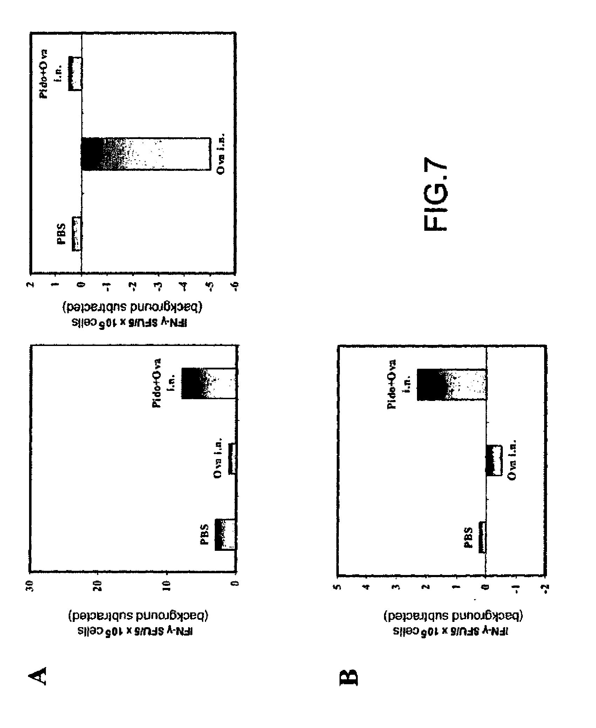

After one (Fig. 7A), and eight (Fig. 7B) weeks from the last immunization, the number of INF-γ-secreting splenocytes was assessed after stimulation with the peptides representing the MHC class I-restricted epitopes. As highlighted in Fig. 7, the number of INF-γ-producing cells after 16-hour stimulation with the dominant and subdominant peptides was significantly higher in samples derived from OVA + Pidotimod-immunized animals intranasally, compared to those immunized with OVA alone. Instead, the oral administration of Pidotimod did not give significant results compared to the administration with OVA alone (non-shown data).

### Production of OVA-specific IgGs by splenocytes and bone marrow cells after immunization with Pidotimod intranasally

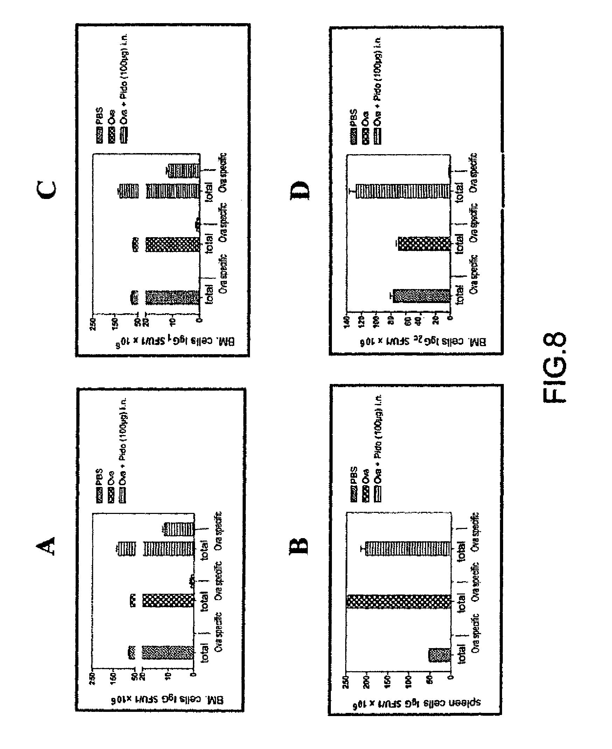

It is known that, following the first meeting with a specific antigen, the immature precursors of plasma cells give rise to a short immune response of a humoral type. Most of the antibodies-secreting cells (ASCs), which are generated after a second meeting with the same antigen, leave the secondary lymphoid organs and lead to the bone marrow, in the lymphoid tissues associated to the mucosal membranes, and in the chronically inflamed tissues. These ASCs take the intrinsic phenotype of the mature plasma cells, and can survive more than one year. Since the half-life of the several subclasses of IgGs is about three weeks, the maintenance upon time of a high antibody titer by bone marrow ASCs is highly important. For this reason, it has been assessed, through ELISPOT assays, the ability of immunized mice medullary cells to secrete total and OVA-specific IgGs after 6 hours from the re-stimulus. The reason for a short-term re-stimulation is to determine exclusively those plasma cells which are already mature and actively secreting. As shown in Fig. 8, in the bone marrow of mice which received OVA + Pidotimod intranasally, the number of both total and OVA-specific IgG- and IgGl - secerning cells, and of the total IgG2s alone was significantly higher than in animals treated only with OVA, while no significant difference was detected in the number of IgG-producing splenocytes. The oral administration of Pidotimod did not give significant results compared to the administration of OVA alone (non-shown data).

### Pidotimod generates a cytotoxic OVA-specific response in vivo

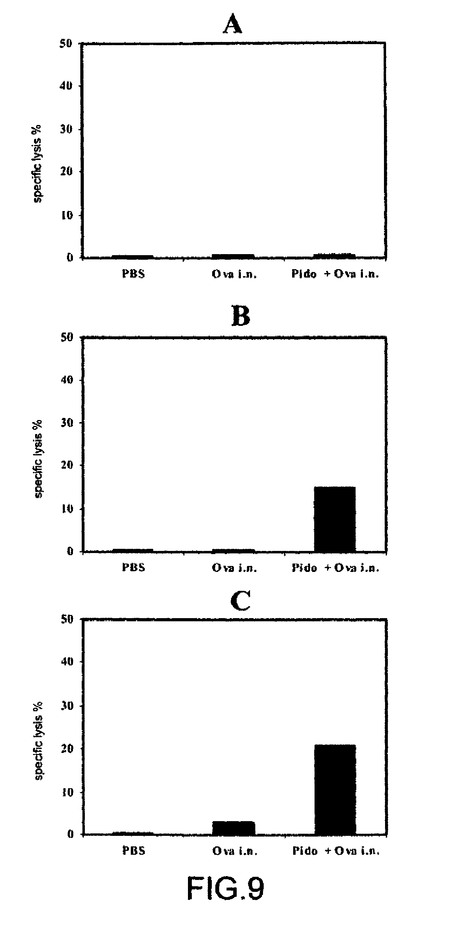

With the aim of assessing the Pidotimod ability to generate a specific cytotoxic response towards Ovalbumin, after 21 days from the last immunization splenocytes of naive mice pulsed with the OVA peptides and marked with CFSE and CMTMR were inoculated, as described above. The ability of immunized mice splenocytes to give origin to a cytotoxic response towards the OVA dominant and cryptic peptide-pulsed cells was higher in OVA + intranasal Pidotimod-treated mice (Fig. 9A and Fig. 9B, respectively), while a negligible lysis percentage was towards the cells pulsed with the subdominant peptide (Fig. 9C). The administration of Pidotimod via oral route did not give significant results compared to the administration of OVA alone (non-shown data).

### Bibliographic references

1. Sallusto F, Lanzavecchia A. 1994. Efficient presentation of soluble antigen by cultured dendritic cells is maintained by granulocyte/macrophage colony-stimulating factor plus interleukin 4 and downregulated by tumor necrosis factor α. J Exp Med, 179: 1109-18.
2. Dauer M, Obermaier B, Herten J, Haerle C, Pohl K5 Rothenfusser S, Schnurr Max, Endres S, Eigler A. 2003. Mature dendritic cells derived from human monocytes within 48 hours: a novel strategy for dendritic cell differentiation from blood precursor. J Immunol, 170: 4069-76.
3. Coppi G, Mailland F5 1990, Immunopharmacolofical studied on PGT/lAm a new immunostimulating drug, Pharmacol Res, 22:16-8.
4. Barchielli M, Coppi G, 1990, Experimental studies on PGT/1A, a new immunostimulating drug, Eur J Pharmaco, 183:909-12.
5. Migliorati G5 D'Adamio L, Coppi G, Nicoletti THE, Riccardi C, 1992, Pidotimod stimulates natural killer cell activity and inhibits thymocyte cell death, Immunopharmacol Immunotoxicol, 14:737-48.

## Claims

1. Use of Pidotimod for the preparation of a medicament in a form which can be administered through the intranasal mucosal route, useful as an adjuvant in enhancing both the humoral, and cell-mediated specific immune response towards a specific antigen.

2. The use of Pidotimod for the preparation of a medicament which can be administered through the intranasal mucosal route, for the prevention of respiratory tract infections, or for the immune response enhancement during respiratory tract infections.

3. The use of Pidotimod for the preparation of a medicament which can be administered through the intranasal mucosal route as an adjuvant of a mucosal vaccine.

4. The use according to any of the claims 1 to 3, wherein said medicament can be administered as a spray or an aerosol.

5. The use according to any of the claims 1 to 4, wherein said medicament is in the form of an aqueous brine solution.

6. The use according to any of the claims 1 to 5, wherein said medicament is in the form of a solution containing 1 mg/liter to 10 g/liter, preferably 500 mg/liter to 5 g/liter, Pidotimod.

7. A pharmaceutical product comprising a mucosal vaccine and Pidotimod in a form which can be administered through the intranasal mucosal route, as a combined preparation for the simultaneous, separated, or sequential use in the prevention of respiratory tract infections.

8. The pharmaceutical product according to claim 7, wherein Pidotimod is in the form of a solution which can be administered as a spray or an aerosol.

9. The pharmaceutical product according to claim 7 or 8, wherein Pidotimod is in the form of a physiological solution.

10. The pharmaceutical product according to any of the claims 7 to 9, wherein Pidotimod is in the form of a solution containing 1 mg/liter to 1 g/liter Pidotimod.
